# EUROPEAN PATENT APPLICATION

(11) **EP 3 881 854 A1**
(43) Date of publication of application: **22.09.2021**
(21) Application number: 19874410.4
(22) Date of filing: 26.09.2019
(51) Int. Cl.: A61K 36/63, A61P 25/00, A23L 33/105

(54) **COMPOSITION COMPRISING PLANT EXTRACT OF GENUS FRAXINUS AS ACTIVE INGREDIENT FOR PREVENTING, ALLEVIATING, OR TREATING SLEEP DISORDERS**

(30) Priority: 17.10.2018 KR 20180123840
(71) Applicant: Korea Institute of Oriental Medicine, Daejeon 34054 (KR)
(72) Inventor: LEE, Mi Young, Seoul 06183 (KR); KIM, Yu Ri, Daejeon 34046 (KR); KIM, Young Hwa, Gwangmyeong-si Gyeonggi-do 14346 (KR); PARK, Bo-Kyung, Daejeon 35251 (KR)
(74) Representative: Nony
(86) International application number: PCT/KR2019/012498
(87) International publication number: WO 2020/080696

(57) **Abstract**

The present invention relates to a composition for preventing, ameliorating, or treating sleep disturbance comprising extract of Fraxinus sp. plant as an effective component. As the extract of *Fraxinus rhynchophylla* of the present invention has an effect of ameliorating sleep disturbance by increasing the mRNA expression level of *calretinin, neuropeptide Y, GAD65* and *GAD67* in an animal model which has sleep disturbance caused by stress, it can be advantageously used as a raw material of a functional health food or a pharmaceutical product for preventing, ameliorating, or treating sleep disturbance.

## Description

### TECHNICAL FIELD

The present invention relates to a composition for preventing, ameliorating, or treating sleep disturbance comprising extract of Fraxinus sp. plant as an effective component.

### BACKGROUND ART

Sleep is a state in which conscious activity is in rest with eyes closed, and it is an important process of restoring the energy consumed during daytime activity and recovering from the fatigue accumulated through physical activities. Sleep is not only a period during which energy restoration and fatigue recovery occur but also a period during which the growth hormone that is essentially required for human growth are secreted in the largest amount.

In human body, the brain governs all physiological functions for sustaining life and, for maintaining a suitably balanced activity, the brain needs a rest, which is mostly achieved during sleep. Due to the overwhelming and busy daily cycle of modern life, increased prevalence in obesity, population aging, and the like, the number of patients who are treated after diagnosis with sleep disturbance has increased in last several years. The number is expected to continue to rise in the coming years.

Among the various types of sleep disturbance, insomnia is one of the most common sleep disturbance and it is defined as a symptom of having difficulty with sleep like difficulty to fall asleep, difficulty to maintain sleep, shallow sleep, or poor sleep quality. Regardless of the stage of insomnia, it is reported that three in ten adults suffer from sleep disturbance and the ratio is even higher in women and seniors.

The reason of having drastically reduced sleep duration by people in the modern world is based on various causes like an increase in mental disorders that are based on psychological reasons such as anxiety about future, depression, anxiety disorder, or stress, alternating day and night shifts resulting from diversity in jobs and society, unhealthy lifestyles, and the like. Namely, the more complex society becomes, the more jobs to be done and the more stress to be dealt with, yielding chronic sleep deficiency. In addition, drinking excess amounts of caffeinated beverages like coffee is also considered to be one reason of having sleep deficiency. Temporary acute insomnia easily occurs due to the irregular sleeping habit caused by temporary stress, change in sleeping habit, or the like. Temporary acute insomnia can be overcome when regular sleeping habit is practiced and underlying issues or stress for causing insomnia are removed so that normal sleeping habit can be restored. However, if a person continues to have a bad sleeping habit or deals with the insomnia in wrongful way, chronic insomnia in which he or she has trouble falling and/or staying asleep every night is caused. Symptoms of chronic insomnia impair the quality of life and increase a risk for depression by 10 times of more. In addition, sleep disturbance caused by insomnia increases the prevalence of various diseases by causing problems in controlling high blood pressure, blood sugar level, obesity or the like, and they also exhibit an influence on social aspect of a patient including higher medical cost, increased risk of having accidents during daytime, poor performance at work, or the like.

Meanwhile, *Fraxinus rhynchophylla* (i.e., Korean ash tree) is naturally found at waterside of foothills and valleys and it can grow to 10 m in height. The bark is grayish brown with irregular patterns having grayish white color. *Fraxinus rhynchophylla* has odd-pinnately compound opposite leaves with 5 to 7 small leaves, which have a wide elliptical shape with length of 6 cm to 15 cm. The leaf has a wavy sawtooth edge and hairs are present on the lower surface leaf vein but not on the upper surface of a leaf. Although the flower is dioecious, some species have a hermaphrodite flower. The flower blooms in May and is borne in panicles at leaf armpit of a young branch. Male flower has two stamens and two calyces while female flower has two to four pistils and two to four calyces, respectively. Flower petal has a upside-down elliptical shape. *Fraxinus rhynchophylla* produces a samara fruit with length of 2 cm to 4 cm, which ripens in September. The samara fruit features an elliptical or an elongated elliptical wing. When *Fraxinus rhynchophylla* branch is immersed in water, the water turns into bluish color so that the Korean name of *Fraxinus rhynchophylla* is "Mool-poo-lae", meaning water turning blue. In Korean traditional medicine, the bark (Fraxini Cortex) is used as a stomachic, an anti-inflammatory agent, or an astringent. *Fraxinus rhynchophylla* is widespread across much of Korea, China, etc.

As a technique relating to extract of *Fraxinus rhynchophylla,* an antimicrobial composition comprising extract of *Fraxinus rhynchophylla* or a compound isolated from the extract is described in Korean Patent Registration No. 0777834. In Korean Patent Registration No. 1820732, a skin whitening composition comprising a fermented product of *Fraxinus rhynchophylla* extract is described. However, so far there is no disclosure of a composition for preventing, ameliorating, or treating sleep disturbance comprising extract of Fraxinus sp. plant as an effective component as it is described in the present invention.

### DETAILED DESCRIPTION OF THE INVENTION

### TECHNICAL PROBLEMS TO BE SOLVED

The present invention is devised under the circumstances that are described above. The present invention relates to a composition for preventing, ameliorating, or treating sleep disturbance comprising extract of Fraxinus sp. plant as an effective component. Specifically, according to the finding that the extract of *Fraxinus rhynchophylla,* which is the effective component of the present invention, has an effect of increasing the mRNA expression level of *calretinin, neuropeptide Y, GAD65* and *GAD67* in an animal model with sleep disturbance caused by stress, the present invention is completed.

### TECHNICAL MEANS FOR SOLVING THE PROBLEMS

To achieve the purpose described above, the present invention provides a pharmaceutical composition for preventing or treating sleep disturbance comprising extract of *Fraxinus rhynchophylla,* which is a Fraxinus sp. plant, as an effective component.

The present invention further provides a functional health food composition for preventing or ameliorating sleep disturbance comprising extract of *Fraxinus rhynchophylla,* which is a Fraxinus sp. plant, as an effective component.

### ADVANTAGEOUS EFFECT OF THE INVENTION

The present invention relates to a pharmaceutical composition for preventing or treating sleep disturbance comprising extract of Fraxinus sp. plant as an effective component. Specifically, the extract of *Fraxinus rhynchophylla,* which is the effective component of the present invention, has an effect of increasing the mRNA expression level of *calretinin, neuropeptide Y, GAD65* and *GAD67* in an animal model which has sleep disturbance caused by stress.

### BRIEF DESCRIPTION OF THE DRAWINGS

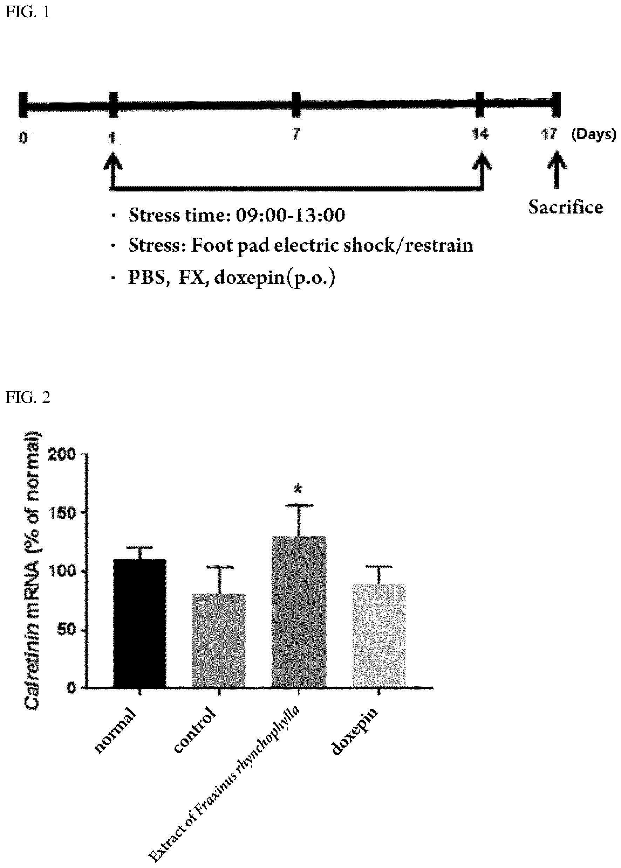

FIG. 1 is a diagram illustrating the process of carrying out a sleep disturbance test, in which the sleep disturbance is caused by foot pad electric shock and restraint stress.
FIG. 2 shows the result of measuring mRNA expression level of *calretinin* in cerebral cortex of a C57BL/6 mouse, which has been induced to have sleep disturbance by foot pad electric shock and restraint stress, in which the cerebral cortex was obtained on Day 17 after administering 100 mg/kg extract of *Fraxinus rhynchophylla* or 15 mg/kg doxepin for 14 days. * indicates that, compared to the control, the mRNA expression level of *calretinin* has increased in statistically significant sense in the group administered with the extract of *Fraxinus rhynchophylla* of the present invention (p<0.05).

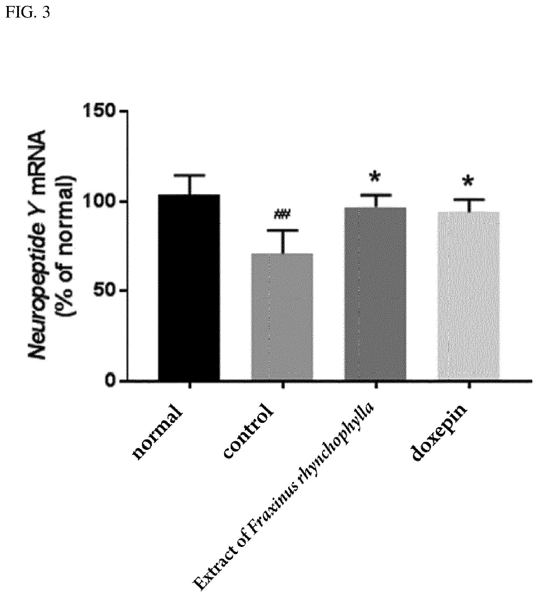

FIG. 3 shows the result of measuring mRNA expression level of *neuropeptide Y* in cerebral cortex of a C57BL/6 mouse, which has been induced to have sleep disturbance by foot pad electric shock and restraint stress, in which the cerebral cortex was obtained on Day 17 after administering 100 mg/kg extract of *Fraxinus rhynchophylla* or 15 mg/kg doxepin for 14 days. ## indicates that, compared to the normal, the mRNA expression level of *neuropeptide Y* has decreased in statistically significant sense in the control (p<0.01). * indicates that, compared to the control, the mRNA expression level of *neuropeptide Y* has increased in statistically significant sense in the group administered with the extract of *Fraxinus rhynchophylla* of the present invention and also in the doxepin group as a positive control (p<0.05).

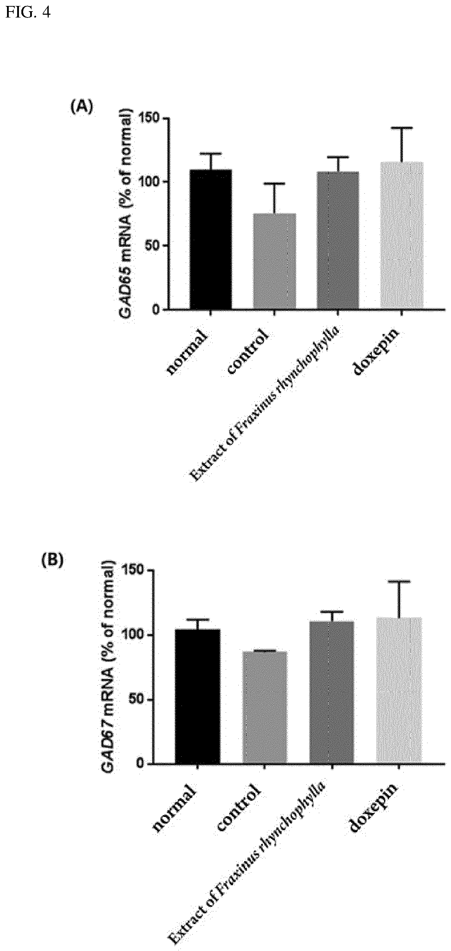

FIG. 4 shows the result of measuring mRNA expression level of *GAD65* (A) and *GAD67* (B) in cerebral cortex of a C57BL/6 mouse, which has been induced to have sleep disturbance by foot pad electric shock and restraint stress, in which the cerebral cortex was obtained on Day 17 after administering 100 mg/kg extract of *Fraxinus rhynchophylla* or 15 mg/kg doxepin for 14 days.

### BEST MODE(S) FOR CARRYING OUT THE INVENTION

The present invention relates to a pharmaceutical composition for preventing or treating sleep disturbance comprising extract of *Fraxinus rhynchophylla* as an effective component.

The extract of *Fraxinus rhynchophylla* can be produced by a method including the following steps:
(1) carrying out extraction by adding an extraction solvent to *Fraxinus rhynchophylla*;
(2) filtering the extract of the step (1); and
(3) concentrating and drying the filtered extract of the step (2) to produce extract,
but the method is not limited thereto.

The extraction solvent of the above step (1) is preferably selected from water, C₁-C₄ lower alcohol, and a mixture thereof. It is more preferably ethanol and even more preferably 70% (v/v) ethanol, but it is not limited thereto. With regard to the production method, any kind of common methods that are generally known as extraction method in the pertinent art, e.g., filtration, hot water extraction, impregnation extraction, extraction by reflux condensation, and ultrasonic extraction, can be used. It is preferable that the extraction is carried out by adding an extraction solvent in an amount of 1 to 20 times the weight of *Fraxinus rhynchophylla.* More preferably, the extraction solvent is added in an amount of 5 to 15 times, and even more preferably added in an amount of 10 times the weight of *Fraxinus rhynchophylla.* The extraction temperature is preferably between 4°C and 100°C, but it is not limited thereto. Furthermore, the extraction time is preferably between 1 hour and 48 hours, more preferably between 1 hour and 24 hours, and most preferably 3 hours, but it is not limited thereto. It is preferable that the concentration of the step (3) in the above method uses a vacuum rotary condenser or a vacuum rotary evaporator, but it is not limited thereto. Furthermore, the drying is preferably carried out by drying under reduced pressure, drying under vacuum, drying under boiling, spray drying, or freeze-drying. It is more preferably freeze-drying, but it is not limited thereto.

The extract of *Fraxinus rhynchophylla* is preferably a Fraxini cortex extract of stem bark or branch bark of *Fraxinus rhynchophylla,* but it is not limited thereto.

The sleep disturbance is preferably insomnia caused by stress, but it is not limited thereto.

The pharmaceutical composition of the present invention may further comprise, in addition to the extract of *Fraxinus rhynchophylla,* a pharmaceutically acceptable carrier, vehicle, or diluent, and the composition may be prepared in various formulations including an oral formulation and a parenteral formulation, but it is not limited thereto.

As for the solid preparation for oral administration, a tablet, a pill, a powder preparation, a granule, a capsule or the like are included, and such solid preparation is produced by mixing at least one compound with one or more vehicles such as starch, calcium carbonate, sucrose, lactose, or gelatin. Furthermore, other than simple vehicles, a lubricating agent such as magnesium stearate or talc is also used. For the liquid preparation for oral administration, a suspension, a solution preparation for internal use, an emulsion, a syrup preparation, or the like can be mentioned. Other than water or liquid paraffin as a commonly used simple diluent, various kinds of a vehicle such as moisturizing agent, sweetening agent, aromatic agent, or preservatives may be included.

Examples of a preparation for parenteral administration include a sterilized aqueous solution, a non-soluble agent, a suspension agent, an emulsion, a freeze-drying agent, and a suppository agent. As a water insoluble solvent or a suspending agent, propylene glycol, polyethylene glycol, or vegetable oil such as olive oil, and injectable ester such as ethylolate can be used. As a base for a suppository, witepsol, macrogol, tween 61, cacao fat, laurin fat, glycerol, gelatin, or the like can be used.

The pharmaceutical composition of the present invention can be administered either orally or parenterally. In case of parenteral administration, it is preferable to choose external application on skin, intraperitoneal, rectal, intravenous, muscular, subcutaneous, endometrium injection, or intracerebroventricular injection, but it is not limited thereto.

The pharmaceutical composition of the present invention is administered in a pharmaceutically effective amount. As described herein, the expression "pharmaceutically effective amount" means an amount sufficient for treating a disorder at reasonable benefit-risk ratio that can be applied for a medical treatment. The effective dose level may be determined based on a type or severeness of a disorder of a patient, activity of a pharmaceutical, sensitivity to a pharmaceutical, administration period, administration route, excretion ratio, time period for therapy, elements including a pharmaceutical used in combination, and other elements that are well known in the medical field. The composition of the present invention can be administered as a separate therapeutic agent, or it can be used in combination with other therapeutic agent. It can be administered in order or simultaneously with a conventional therapeutic agent. It can be also administered as single-dose or multi-dose. It is important to administer an amount which allows obtainment of the maximum effect with minimum dose while considering all of the aforementioned elements without having any side effect, and the dosage can be easily determined by a person skilled in the pertinent art.

The dosage of the composition of the present invention may vary depending on bodyweight, age, sex, health state, diet of a patient, administration period, administration method, excretion rate, and severeness of disorder. However, the daily dosage is, in terms of the amount of extract of *Fraxinus rhynchophylla,* 0.01 to 1,000 mg/kg, preferably 30 to 500 mg/kg, and more preferably 50 to 300 mg/kg, and it can be administered 1 to 6 times per day. However, since the dosage may be increased or reduced depending on the administration route, severeness of obesity, sex, body weight, age or the like, the scope of the present invention is not limited by the aforementioned dosage in any sense.

The present invention further relates to a functional health food composition for preventing or ameliorating sleep disturbance comprising extract of *Fraxinus rhynchophylla* as an effective component.

The functional health food composition of the present invention is preferably produced in any one formulation selected from a powder, a granule, a pill, a tablet, a capsule, a candy, a syrup, and a drink, but it is not limited thereto.

The functional health food composition of the present invention comprising extract of *Fraxinus rhynchophylla* as an effective component may be directly added to a food product or used with other food product or food ingredient, and it can be suitably used according to a common method. The mixing amount of the effective component can be suitably determined based on the purpose of use (i.e., prevention or amelioration). In general, the amount of extract of *Fraxinus rhynchophylla* to be comprised in the functional health food composition can be 0.1 to 90 parts by weight relative to the total weight of the functional health food composition. However, in case of long-term consumption under the purpose of maintaining good health and hygiene or managing health, it can be an amount below the aforementioned range, and, as there is no problem in terms of safety, the effective component may be also used in an amount above the aforementioned range.

When the functional health food composition of the present invention is consumed in the form of a beverage, other ingredients are not particularly limited except that, as an essential ingredient, the aforementioned extract of *Fraxinus rhynchophylla* is comprised at indicated ratio, and, like common beverages, various flavors or natural carbohydrates can be comprised as an additional component. Examples of the natural carbohydrates include monosaccharides such as glucose or fructose, disaccharides such as maltose or sucrose, polysaccharides such as dextrin or cyclodextrin, and sugar alcohols such as xylitol, sorbitol, or erythritol. As a flavor other than those described above, natural flavor (taumatin, stevia extract (e.g., rebaudioside A and glycyrrhizin)) and synthetic flavor (e.g., saccharine and aspartame) can be advantageously used.

The functional health food composition of the present invention may further comprise, in addition to the effective component, at least one selected from a nutritional supplement, a vitamin, an electrolyte, a flavor, a coloring agent, an enhancing agent, pectinic acid and a salt thereof, alginic acid and a salt thereof, an organic acid, a protective colloidal thickening agent, a pH adjusting agent, a stabilizer, a preservative, glycerin, alcohol, and a carbonating agent used for carbonated drink. Other than those, fruit flesh for producing natural fruit juice or vegetable drink can be comprised in the functional health food composition of the present invention. The fruit flesh may be used either independently or in combination thereof. Ratio of the above various additives is not critical, but it is generally selected from a range of about 0.1 to 20 parts by weight relative to 100 parts by weight of the extract of *Fraxinus rhynchophylla* of the present invention.

Hereinbelow, the present invention is explained in greater detail in view of the Examples. However, the following Examples are given only for specific explanation of the present invention and it would be evident to a person who has common knowledge in the pertinent art that the scope of the present invention is not limited by them.

### EXAMPLES

### Production, preparation, and administration of test sample

Tree bark of *Fraxinus rhynchophylla* was subjected to reflux extraction for 3 hours according to addition into 70% (v/v) ethanol solvent which is 10 to 15 times the weight of the bark. After the first extraction using a filter net, the extracted solvent was subjected to the second cotton wool filtration for concentration followed by freeze-drying and pulverization to give the product in powder form.

100 mg/kg extract of *Fraxinus rhynchophylla* and 15 mg/kg doxepin (Sigma-Aldrich, St. Louis, MO, USA) were dissolved respectively in PBS to the designated concentration. After aliquoting them according to the required administration amount, it was orally administered in an amount of 0.1 ml for each, one hour before applying stress. To the normal and stress control, PBS was orally administered in an amount of 0.1 ml for each.

### Animal model with sleep disturbance induced by stress

Seven-week old male C57BL/6 mouse with bodyweight of 20 to 22 g was obtained from DBL Co., Ltd. (Eumseong-Gun, Chungcheong-Bukdo, Korea). The animal was supplied with a sufficient amount of water and solid feed (not added with any antibiotics, Samyang Animal Feed Co.) till the test day, and it was used for the experiment after acclimation for 1 week under an environment with temperature of 22±2°C, humidity of 55±15%, and 12-hour light and dark cycle.

The experiment was carried out as illustrated in FIG. 1. For 14 days in total, foot pad electric shock and restraint stress experiment was carried out every day between AM 09:00 and PM 01:00. To induce sleep disturbance, electric shock was applied, at 0.5 mA, for 2 minutes (for 1 second per 10 seconds) to the foot pad of a mouse by using a shuttle box (JEUNG DO BIO & PLANT CO, LTD, Seoul, Korea), and then the mouse was restrained for 2 hours using an acrylic hemi-cylindric restraint cage (JEUNG DO BIO & PLANT CO, LTD, Seoul, Korea).

### Measurement of mRNA expression level of calretinin, neuropeptide Y, GAD65, and GAD67 in cerebral cortex

Expression pattern of the gene in cerebral cortex, which has been removed from each test animal upon the completion of the test, was determined by real-time PCR. The cerebral cortex tissues of a mouse were treated with RNAzolB (Tel-Test) to extract RNA, and then analyzed by cDNA and real-time PCR instrument using One-step SYBR Green PCR kit (AB science). The cerebral cortex tissues were added with 500 µl of RNAzol(B), disrupted using a homogenizer, and then added with 50 µl of chloroform (CHCl₃) followed by mixing again for 15 seconds. The resultant was allowed to stand on ice for 15 minutes and centrifuged at 13,000 rpm. Accordingly, the supernatant in an amount of about 200 µl was obtained and admixed with 2-propanol (200 µl) followed by mild shaking. The mixture was allowed to stand on ice for 15 minutes. After centrifuge again at 13,000 rpm, the resultant was washed with 80% ethanol and dried for 3 minutes in vacuum pump to extract RNA. The extracted RNA was dissolved in 20 µl distilled water which has been treated with diethyl pyrocarbonate (DEPC), and, after the inactivation on a heating block at 75°C, used for the synthesis of first strand cDNA. For reverse transcription, the prepared total RNA (3 µg) was reacted with DNaseI (10 U/µl) 2U/tube for 30 minutes on a 37°C heating block followed by denaturation for 10 minutes at 75°C. After adding 2.5 µl of 10 mM dNTPs mix, 1 µl of random sequence hexanucleotides (25 pmole/25 µl), and 1 µl of RNase inhibitor (20 U/µl) as an RNA inhibitor, 1 µl of 100 mM DTT, and 4.5 µl or 5×RT buffer (250 mM Tris-HCl, pH 8.3, 375 mM KCl, 15 mM MgCl₂), 1 µl of M-MLV RT (200 U/µl) was added again to the mixture, which was then adjusted to have final volume of 20 µl using DEPC-treated distilled water. The resulting reaction mixture (20 µl) was thoroughly mixed and then subjected to centrifugal precipitation at 2,000 rpm for 5 seconds. After the reaction for 45 minutes on 37°C heating block, first-strand cDNA was synthesized, which was then allowed to stand at 95°C for 5 minutes to inactivate M-MLV RT. Thus-obtained cDNA after complete synthesis was used for PCR (polymerase chain reaction). Real time quantitative PCR was carried out by using Applied Biosystems 7500 Real-Time PCR system (Applied Biosystems, USA).

**Table 1**

| Primer sequences for *calretinin, neuropeptide Y, GAD65,* and *GAD67* | | |
|---|---|---|
| Gene | | Primer sequence |
| Calretinin | forward (SEQ ID NO: 1) | 5'-CTAAGCTCCAGGAGTACACC-3' |
| | reverse (SEQ ID NO: 2) | 5'-GCATTGAACTCTTCTGAGGTC-3' |
| Neuropeptide Y | forward (SEQ ID NO: 3) | 5'-AGGCTTGAAGACCCTTCCAT-3' |
| | reverse (SEQ ID NO: 4) | 5'-ACAGGCAGACTGGTTTCAGG-3' |
| GAD65 | forward (SEQ ID NO: 5) | 5'-TCAACTAAGTCCCACCCTAAG-3' |
| | reverse (SEQ ID NO: 6) | 5'-CCCTGTAGAGTCAATACCTGC-3' |
| GAD67 | forward (SEQ ID NO: 7) | 5'-CTCAGGCTGTATGTCAGATGTTC-3' |
| | reverse (SEQ ID NO: 8) | 5'-AAGCGAGTCACAGAGATTGGTC-3' |
| GAPDH | forward (SEQ ID NO: 9) | 5'-AAGGTGGTGAAGCAGGCAT-3' |
| | reverse (SEQ ID NO: 10) | 5'-GGTCCAGGGTTTCTTACTCCT-3' |

### <Statistical treatment>

The results are given in mean±standard deviation, and the statistical comparison among test groups was achieved by carrying out one-way measures analysis of variance (ANONA) based on Tukey's Honest Significant Difference (HSD). *p*<0.05 was taken to have statistical significance.

### Example 1. Determination of effect of Fraxinus sp. extract on mRNA expression level of calcium binding protein in C57BL/6 mouse induced to have sleep disturbance

To examine any influence of the extract of *Fraxinus rhynchophylla* exhibited on the mRNA expression level of *calretinin* in cerebral cortex of C57BL/6 mouse which has been induced to have sleep disturbance, PBS was orally administered, according to the aforementioned experimental method, to a C57BL/6 mouse induced to have sleep disturbance for the normal and stress control. The positive control was administered with 15 mg/kg doxepin and the extract administration group was administered with 100 mg/kg extract of *Fraxinus rhynchophylla.* After 1 hour, foot pad electric shock and restrain were applied to the mouse to induce sleep disturbance for 14 days. Cerebral cortex was collected by autopsy of the mouse to obtain RNA. cDNA was then prepared and mRNA expression of *calretinin* was analyzed by using real-time gene analyzer.

The result indicates that, as shown in FIG. 2, the mRNA expression level of *calretinin* tends to be lower in the control compared to the normal. The group administered with the extract of *Fraxinus rhynchophylla* showed higher expression level than the control in statistically significant sense.

### Example 2. Determination of effect of Fraxinus sp. extract on mRNA expression level of neuronal protein, which is expressed in GABAergic neurons, in C57BL/6 mouse induced to have sleep disturbance

To examine any influence of the extract of *Fraxinus rhynchophylla* exhibited on the mRNA expression of *neuropeptide Y* in cerebral cortex of C57BL/6 mouse which has been induced to have sleep disturbance, test samples were orally administered for 14 days according to the experimental method described above. Thereafter, foot pad electric shock and restrain were applied to the mouse and then cerebral cortex was collected by autopsy of the mouse to obtain RNA. cDNA was then prepared and mRNA expression of *neuropeptide Y* was analyzed by using real-time gene analyzer.

The result indicates that, as shown in FIG. 3, the mRNA expression of *neuropeptide Y* is lower in the control in significant sense. The group administered with the extract of *Fraxinus rhynchophylla* and the group administered with the positive control showed higher expression in significant sense.

### Example 3. Determination of effect of Fraxinus sp. extract on mRNA expression of GABA-synthesizing enzyme in C57BL/6 mouse induced to have sleep disturbance

To examine any influence of the extract of *Fraxinus rhynchophylla* exhibited on the mRNA expression of *GAD65* (glutamic acid decarboxylase 65) and *GAD67* (glutamic acid decarboxylase 67) in cerebral cortex of C57BL/6 mouse which has been induced to have sleep disturbance, test samples were orally administered for 14 days according to the experimental method described above. Thereafter, foot pad electric shock and restrain were applied to the mouse and then cerebral cortex was collected by autopsy of the mouse to obtain RNA. cDNA was then prepared and mRNA expression level of *GAD65* and *GAD67* was analyzed by using real-time gene analyzer.

The result indicates that, as shown in FIG. 4, the mRNA expression level of *GAD65* and *GAD67* tends to be lower in the control. On the other hand, the group administered with the extract of *Fraxinus rhynchophylla* and the group administered with the positive control tend to show higher expression.

## Claims

1. A pharmaceutical composition for preventing or treating sleep disturbance comprising extract of *Fraxinus rhynchophylla* as an effective component.

2. The pharmaceutical composition for preventing or treating sleep disturbance according to Claim 1, wherein a solvent for extracting the extract of *Fraxinus rhynchophylla* is water, C₁-C₄ lower alcohol, or a mixture thereof.

3. The pharmaceutical composition for preventing or treating sleep disturbance according to Claim 1, wherein the sleep disturbance is insomnia caused by stress.

4. The pharmaceutical composition for preventing or treating sleep disturbance according to Claim 1, wherein the composition further comprises a pharmaceutically acceptable carrier, vehicle, or diluent in addition to the extract of *Fraxinus rhynchophylla.*

5. A functional health food composition for preventing or ameliorating sleep disturbance comprising extract of *Fraxinus rhynchophylla* as an effective component.

6. The functional health food composition for preventing or ameliorating sleep disturbance according to Claim 5, wherein the composition is produced in any one formulation selected from a powder, a granule, a pill, a tablet, a capsule, a candy, a syrup, and a drink.


<110>     Korea Institute of Oriental Medicine

<120>     Composition for preventing, ameliorating or treating sleep
          disturbance comprising extract of Fraxinus sp. plant as effective
          component

<130>     PCT1930

<160>     10

<170>     KopatentIn 2.0

<210>     1
<211>     20
<212>     DNA
<213>     Artificial Sequence

<220>
<223>     Calretinin-F


<400>     1
ctaagctcca ggagtacacc                                                   20


<210>     2
<211>     21
<212>     DNA
<213>     Artificial Sequence

<220>
<223>     Calretinin-R


<400>     2
gcattgaact cttctgaggt c                                                 21


<210>     3
<211>     20
<212>     DNA
<213>     Artificial Sequence

<220>
<223>     Neuropeptide Y-F


<400>     3
aggcttgaag acccttccat                                                   20


<210>     4
<211>     20
<212>     DNA
<213>     Artificial Sequence

<220>
<223>     Neuropeptide Y-R


<400>     4
acaggcagac tggtttcagg                                                   20

<210>    5
<211>    21
<212>    DNA
<213>    Artificial Sequence

<220>
<223>    GAD65-F


<400>    5
tcaactaagt cccaccctaa g                                                21


<210>    6
<211>    21
<212>    DNA
<213>    Artificial Sequence

<220>
<223>    GAD65-R


<400>    6
ccctgtagag tcaatacctg c                                                21


<210>    7
<211>    23
<212>    DNA
<213>    Artificial Sequence

<220>
<223>    GAD67-F


<400>    7
ctcaggctgt atgtcagatg ttc                                              23


<210>    8
<211>    22
<212>    DNA
<213>    Artificial Sequence

<220>
<223>    GAD67-R


<400>    8
aagcgagtca cagagattgg tc                                               22


<210>    9
<211>    19
<212>    DNA
<213>    Artificial Sequence

<220>
<223>    GAPDH-F


<400>    9

aaggtggtga agcaggcat 19

<210> 10
<211> 21
<212> DNA
<213> Artificial Sequence

<220>
<223> GAPDH-R

<400> 10
ggtccagggt ttcttactcc t 21